# EUROPEAN PATENT SPECIFICATION

(11) **EP 0 288 901 B1**
(45) Date of publication and mention of the grant of the patent: **10.02.1993**
(21) Application number: 88106368.9
(22) Date of filing: 21.04.1988
(51) Int. Cl.: C12N 15/53, C12N 9/08

(54) **Recombinant human glutathione peroxidase, process for preparing the same and DNA coding the same**
Rekombinante menschliche Glutathionperoxidase, Verfahren zur Herstellung des Enzyms und dafür kodierende DNA
Glutathion peroxydase humaine recombinante, procédé de préparation et ADN codant pour cet enzyme

(30) Priority: 25.04.1987 JP 102383/87
(43) Date of publication of application: 02.11.1988
(73) Proprietor: NIPPON KAYAKU KABUSHIKI KAISHA, Tokyo 102 (JP)
(72) Inventor: Sukenaga, Yoshikazu, Nerima-ku Tokyo (JP); Ishida, Koichi, Kita-ku Tokyo (JP); Takeda, Teruyo, Meguro-ku Tokyo (JP); Takagi, Kenkichi, Nerima-ku Tokyo (JP)
(74) Representative: Türk, Gille, Hrabal, Leifert

(56) References cited:
- WO-A-88/07541
- NUCLEIC ACIDS RESEARCH, vol. 15, no. 17, 1987, page 7178, IRL Press Ltd, Oxford, GB; Y. SUKENAGA et al.: "cDNA sequence coding for human glutathione peroxidase"
- CHEMICAL ABSTRACTS, vol. 105, no. 9, 1st September 1986, page 168, abstract no. 73610v, Columbus, Ohio, US; I. CHAMBERS et al.: "The structure of the mouse glutathione peroxidase gene: the selenocysteine in the active site is encoded by the 'termination' codon, TGA", & EMBO J. 1986, 5(6), 1221-7

## Description

### Field of the invention:

This invention relates to a DNA coding for recombinant human glutathione peroxidase, a plasmid containing said DNA and a process for preparing said peroxidase.

Recently it has been believed that an increase in lipid peroxides is causative of various diseases such as hepatic disorders, myocardial infraction, inflammation, scabies, radiation hazard, arteriosclerosis, cerebral apoplexy, diabetes or cancer. Therefore the application of glutathione peroxidase to medical purposes has been more and more extending. For example, this polypeptide is employed in the analysis of lipid peroxides or the treatment and/or prophylaxis of diseases caused by peroxides such as hydrogen peroxide of lipid peroxides induced by active oxygen.

Glutathione peroxidase was found by G. C. Mills for the first time in bovine erythrocytes (J. Biol, Chem., 229, 189 (1957)). A subsequent report described that it was found in other organs such as liver (Arch. Biochem. Biophys., 86, 1 (1960)). Further there have been reported a total amino acid sequence of this polypeptide and a DNA base sequence coding the same in mice (The EMBO Journal, 5 (6), 1221 - 1227 (1986)); a total amino acid sequence thereof in cattle (Hoppe-Seylers Z, Physiol. Chem., 365, 195 - 212 (1984); and a partial amino acid sequence thereof in rats (Biochem. Biophys. Acts, 709, 304 - 309 (1982)). It is known that glutathione peroxidase occurs in almost all human organs including kidney, lung, brain, heart and main artery. It has been further reported that this polypeptide taken from a human erythrocyte is a tetramer having a molecular weight of 95,000 (J. Biol. Chem., 250, 5144 (1975)). However neither the amino acid sequence of this polypeptide nor the DNA base sequence coding the same has been published before.

Therefore it has been required to develop a process for readily preparing glutathione peroxidase originating from human on a large scale from the viewpoint of, for example, antigenicity.

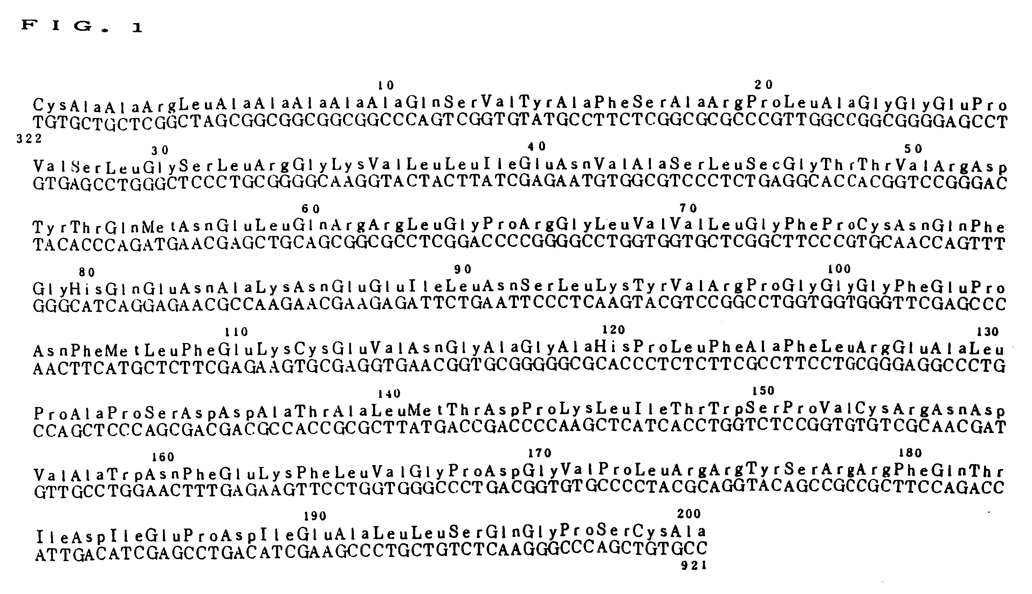

The present invention relates to a DNA having the base sequence as shown in Fig. 1 and coding the amino acid sequence of recombinant human glutathione peroxidase, which will be abbreviated to recombinant hGSH·Px hereinafter, a plasmid containing said DNA and a process for preparing the recombinant hGSH·Px which comprises manifesting said DNA in a recombination host.

### Brief Description of the Drawings:

Fig. 1 shows the amino acid sequence of recombinant hGSH·Px and the base sequence of a DNA coding the same.

Fig. 2 shows the whole base sequence of the cDNA of hGSH·Px.

Fig. 3 is a restriction map of pHGP 7.

Fig. 4 is a restriction map of pSPHGP 7.

Fig. 5 shows a process for preparing ptacHGP 7 and a restriction map thereof.

### Detailed Description of the Invention:

The term "human glutathione peroxidase", which will be abbreviated to hGSH·Px hereinafter, as used herein means a protein which is produced in the human body and decomposes a peroxide such as hydrogen peroxide or a lipid peroxide at least in vivo according to, for example, the following reaction:

2GSH + ROOH → GSSG + ROH + H₂O.

The term "recombinant hGSH·Px" as used herein includes not only a polypeptide which is produced in a microbial incubation system or a cell incubation system and has the amino acid sequence of hGSH·Px as shown in Fig. 1, but also a polypeptide comprising the same.

A polypeptide having the above-defined amino acid sequence of hGSH·Px is one which contains another polypeptide bound to the N- or C-terminal of the hGSH·Px polypeptide and shows an activity comparable to that of the hGSH·Px polypeptide.

The present invention involves not only the DNA of Fig. 1 encoding for a polypeptide having the amino acid sequence of hGSH:Px or a vector wherein said DNA is incorporated, and a host transformed with said vector, but also any polypeptide which is highly homologous with the hGSH·Px polypeptide of Fig. 1. Thus the present invention involves, for example, a hGSH·Px DNA derivative of Fig. 1 obtained through partial modification of the gene code as well as a polypeptide obtained therefrom. The term "hGSH·Px DNA derivative obtained by partial modification" means a derivative wherein some portion of the base sequency of hGSH·Px DNA of Fig. 1 is deleted or another DNA fragment is bound to the hGSH·Px DNA.

The following abbreviations are used hereinbelow:
- DNA:: deoxyribonucleic acid,
- A:: adenine,
- I:: inosine,
- T:: thymine,
- G:: guanine,
- C:: cytosine,
- RNA:: ribonucleic acid,
- dATP:: deoxyadenosine triphosphate,
- dGTP:: deoxyguanosine triphosphate,
- dCTP:: deoxycytidine triphosphate,
- TTP:: thymidine triphosphate,
- ATP:: adenosine triphosphate,
- GTP:: guanosine triphosphate,
- CTP:: cytidine triphosphate,
- UTP:: uridine triphosphate,
- Gly:: glycine,
- Ala:: alanine,
- Vrl:: valine
- Leu:: leucine,
- Ile:: isoleucine,
- Ser:: serine,
- Thr:: threonine,
- Cys:: cysteine,
- Sec:: selenocysteine,
- Glu:: glutamic acid,
- Asp:: aspartic acid
- Lys:: lysine,
- Arg:: arginine,
- His:: histidine,
- Phe:: phenylalanine,
- Tip:: tryptophan,
- Pro:: proline,
- Asn:: asparagine,
- Gln:: glutamine,
- Tris:: tris hydrochloride,
- Kbp:: kilobase pair,
- bp:: base pair,
- in vitro:: out of a living organism,
- in vivo:: within a living organism,
- cDNA:: complementary DNA
- Amp^{R}:: ampicillin-resistant gene,
- Amp:: ampicillin,
- GSH:: reduced glutathione,
- ROOH: peroxide,
- GSSG:: oxidized glutathione,
- Tyr:: tyrosine,
- ROH:: hydroxide, and
- Met:: methionine.

Fig. 1 shows the amino acid sequence of the recombinant hGSH·Px of the present invention and the base sequence of a DNA coding the same. This recombinant hGSH·Px may be obtained by manifesting a DNA obtained from human liver mRNA;
Based on the findings on the amino acid sequence of the hGSH·Px polypeptide and DNA base sequence as clarified by the present invention, a DNA coding a polypeptide having the amino acid sequence of hGSH·Px as shown in Fig. 1 or a partially modified variant of hGSH·Px can be obtained through conventional gene recombination techniques. Further a recombinant hGSH·Px activity can be readily produced by using the same. It is further possible to conduct total synthesis of a DNA having the base sequence of Fig. 1 coding the amino acid sequence of the hGSH·Px polypeptide based thereon. The present invention involves all of those described above.

A DNA having the base sequence of the present invention coding for the amino acid sequence of the recombinant hGSH·Px can be prepared by, for example, the following process.

### (1) Preparation of probe for detecting cloned DNA coding hGSH·Px

A single-stranded DNA of an appropriate length corresponding to the DNA sequence of an appropriate site of mouse glutathione peroxidase (mouse GSH·Px) as reported by I. Chambers et al. (The EMBO J., 5 (6), 1221 - 1227 (1986)) is chemically synthesized and the 5'-terminal thereof is labeled with ³²P to thereby give a probe for cloning hGSH.Px cDNA as will be described hereinbelow.

### (2) Preparation of human liver mRNA

All of the RNAs contained in a human liver tissue are extracted by a conventional method such as guanidine thiocyanate method (Biochem., 18, 5294 - 5299 (1979)), and treated in a conventional manner by using, for example, an oligo (dT) cellulose column to thereby separate a poly(A) mRNA fraction.

### (3) Preparation of human liver cDNA library

By using the mRNA obtained in the step (2) as a template, a plasmid wherein cDNA is incorporated is prepared according to, for example, Okayama-Berg's method (Mol. Cell Biol., 2, 161 - 170 (1982)). Alternately, the mRNA may be converted into a single-stranded cDNA by using reverse transcriptase in a conventional manner. Then the obtained single-stranded cDNA may be converted into a double-stranded DNA, which is then incorporated into a plasmid. The recombinant plasmid thus obtained is then introduced into a host such as E. coli X 1776 strain according to, for example, a method reported by Maniatis et al. (Molecular Cloning, Cold Spring Harber Laboratory, 254 - 255 (1982)) to thereby transform the host. By selecting ampicillin-resistant strains, a cDNA library is obtained.

### (4) Cloning of hGSH·Px cDNA

Clones to be hybridized with a mouse GSH·Px DNA fragment are screened by subjecting the cDNA library obtained in the step (3) to a colony hybridization test by using the single-stranded DNA fragment coding mouse GSH·Px obtained in the step (1) as a probe.

The base sequences of the cloned DNAs thus obtained are analyzed according to, for example, M13 dideoxy method (Proc. Natl. Acad. Sci. U.S.A., 74 5463 - 5467 (1977)). Thus base sequences homologous with that of mouse GSH·Px which has been already clarified are detected and a cDNA having a base sequence corresponding to the whole translation region is finally selected to thereby give a cloned DNA having a base sequence coding the hGSH·Px polypeptide.

The hGSH·Px polypeptide can be obtained by excising the region coding hGSH·Px of the cloned DNA of the present invention, incorporating the incised region into a gene expression vector having an appropriate promoter, introducing said vector into a host such as E. coli C 600 and purifying the aimed protein with the guidance of the hGSH·Px activity.

Examples of said promoter include lac promoter, trp promoter, tac promoter, PL promoter and SV 40 primary promoter. As the vector, any one capable of growing in a host to be transformed may be employed. For example, a plasmid such as pBR322, a phage such as λ phage or a virus such as SV40 are available. One of these vectors may be employed alone. Alternately, a combination thereof, for example, pBR322-SV 40 hybrid plasmid may be used. The vector wherein the cloned DNA is incorporated is introduced into a host to thereby transform the same. Thus a gene expression host can be obtained. As the host to be transformed, a microorganism such as E. coli, Bacillus subtilits, a yeast or an animal- or vegetable-culture cell such as cos cell may be used.

The gene expression host may be incubated in a conventional manner suitable for the host. Since hGSH·Px comprises cystein, which is an amino acid containing selenium, as one of its constituting amino acids, it is preferable to add a selenium source such as selenous acid to the medium at a ratio of approximately 10 µM to 2 mM, preferably approximately 20 µM to 1 mM. After the completion of the incubation, the cells are collected and ground. The aimed hGSH·Px may be obtained from the supernatant of the ground cells.

hGSH·Px may be obtained from the supernatant at a high purity by, for example, the following method. The supernatant is treated with, for example, a DEAE cellulose exchanges column to thereby remove strongly acidic components including nucleic acid therefrom through absorption. The eluate thus obtained is dialyzed against a phosphate buffer free from common salt, adsorbed by, for example, a DEAE cellulose exchanges column and then subjected to gradient elution with common salt. The hGSH·Px fraction is concentrated by ultrafiltration and then passed through, for example, a gel filtration column such as Sephadex G100. The obtained hGSH·Px fraction is further concentrated and then passed through, for example, a preparative SDS acrylamide gel. Thus hGSH·Px can be obtained at a high purity.

### Example

### (1) Preparation of probe for detecting cloned DNA coding hGSH·Px

A single-stranded DNA containing inosine (I) in part represented by the following formula, which was complementary to some portion of the base sequence of a DNA coding mouse GSH·Px as reported by I. Chambers et al., was chemically synthesized: 
(5')-GTGGTGCCTCAGAGIGAIGCGACITTCTCI
ATGAGCAGCACCTTGCCICGCAGGGA-(3')
This single-stranded DNA comprising 56 bases was labeled with ³²P and used as a proble for cloning hGSH·Px cDNA in the following steps (6) and (7).

### (2) Isolation of mRNA from human liver

2 g of a fresh human liver tissue was washed with a phosphate-buffered saline (PBS) and all of the RNAs contained in the cytoplasm were extracted therefrom with guanidine thiocyanate method (Biochem., 18, 5294 - 5299 (1979)).

The whole RNAs thus extracted were dissolved in a buffer of a high salt concentration (Tris containing 0.5 M of NaCl, pH 7.4) and the resulting solution was passed through an oligo (dT) cellulose column. Poly A RNA (mRNA) thus adsorbed was eluted with a buffer of a low salt concentration (Tris free from NaCl, pH 7.4) and then precipitated from ethanol. Thus 30 µg of mRNA was obtained from 3 mg of the whole RNAs.

### (3) Annealing of mRNA and synthesis of cDNA

The fraction obtained in the step (2) was treated according to Okayama-Berg's method ('83 Mol. Cell Biol., 3, 280 (1983)) in the following manner. Namely, 20 µl of a solution containing 50 mM of Tris (pH 8.3), 30 mM of KCl, 0.3 mM of dithiothreitol (DTT), 8 mM of MgCl, 40 µg/ml of actinomycin D, 2 mM portions of dATP, dCTP, dGTP and TTP, 30 µCi[α-³²P] dCTP (600 Ci/mmol), 280 U ribonuclease inhibitor and 3.8 µg of plasmid primer, which was a primer of T-tailing comprising approximately 60 bases and synthesized by using E. coli plasmid pcDvl (a product of Pharmacia) DNA for Okayama-Berg primer according to Okayama-Berg's method, was prepared and maintained at 37°C.

Then 20 µl of a solution containing 10 mM of Tris (pH 8.0), 1 mM of EDTA and 3 µg of mRNA was prepared and maintained at 65°C for five minutes. Then it was immediately cooled to 37°C and mixed with 20 µl of the abovementioned solution. The resulting mixture was maintained at this temperature for additional five minutes. Then 20 U of reverse transcriptase was added thereto and the mixture was heated to 37°C for 20 minutes. Subsequently the reaction was ceased by adding 4 µl of 250 mM EDTA and 2 µl of a 10 % SDS solution thereto. The reaction mixture was extracted with phenol/chloroform (1 : 1) and precipitated from ethanol twice.

### (4) Preparation of recombinant plasmid

The precipitate obtained in the step (3) was dissolved in a solution containing 140 mM of sodium cacodylate, 30 mM of Tris (pH 6.8), 1 mM of CoCl₂, 0.1 mM of DTT, 1 mM of dCTP and 50 µCi [α - ³²P] dCTP and the resulting solution was heated to 37°C for two to three minutes. Then 54 U of terminal deoxynucleotidyl transferase was added thereto to give a total volume of 50 µl. The resulting mixture was heated to 37°C for three minutes and treated in the same manner as the one employed in the above reverse transcription to thereby give a precipitate from ethanol.

This precipitate was dissolved in a solution containing 50 mM of NaCl, mM of Tris (pH 8.0), 10 mM of MgCl₂, 100 g/ml of bovine serum albumin (BSA) and 12 U of Hind III and the resulting solution was heated to 37°C for two to four hours.

Then it was extracted with phenol/chloroform (1 : 1) and precipitated from ethanol. The precipitate thus obtained was dissolved in 20 µl of a solution containing 10 mM of Tris (pH 7.3) and 1 mM of EDTA and 6 µl of ethanol was further added thereto to thereby give a total volume of 26 µl.

To 1 µl of this solution are added 1 µl of a 10-fold concentrate of a solution containing 0.04 pmol of oligo (dG) linker, which was a linker of dG-tailing comprising approximately 12 bases and synthesized by using E. coli plasmid pL1 (a product of Pharmacia) DNA for Okayama-Berg linker according to Okayama-Berg's method, 10 mM of Tris (pH 7.5), 0.1 M of NaCl and 1 mM of EDTA and 8 µl of distilled water to thereby give a total volume of 10 µl. The resulting solution was heated to 65°C for five minutes and then to 42°C for 30 minutes. Subsequently it was maintained at 0°C. Then a ccncentrated solution containing 20 mM of Tris (pH 7.5), 4 mM of MgCl₂, 10 mM of ammonium sulfate, 0.1 M of KCl, 50 µg/ml of BSA, 0.1 mM of β-nicotinamide adenine dinucleotide (β-NAD) and 0.6 µg of E. coli DNA ligase was added thereto to give a final volume of 150 pl. The obtained solution was maintained at 12°C overnight. Then 0.4 µl of a solution containing 20 mM portions of dATP, dGTP, dCTP and TTP, 1 µl of 1.5 mM β-NAD, 0.4 µg of E. coli DNA ligase, 0.3 µg of E. coli DNA polymerase and 1 U of E. coli ribonuclease H was added thereto to give a total volume of 104 µl. The resulting mixture was heated to 12°C for an hour and to 25°C for additional one hour.

The residual solution (25 µl) was also treated in the same manner.

### (5) Transfromation of E. coli

Competent cells were prepared by using E. coli X 1776 strain according to the method reported by Maniatis et al. (Molecular Cloning, Cold Spring Harber Laboratory, 254 - 255 (1982)). 0.2 ml portions thereof were pipetted. 135 lots (each 20 µl) of the DNA solution obtained in the step (2) were transformed. Each transformant thus obtained was suspended in a liquid medium (Amp L medium) containing 10 g/l of bactotrypton, 5 g/l of yeast extract, 5 g/l of sodium chloride, 50 µg/l of ampicillin (Amp), 0.1 g/l of diaminopimelic acid and 0.04 g/l of thymidine and incubated therein at 37°C for 20 hours under shaking. Then it was stored in the form of a 15 % solution in glycerol at - 80°C. Separately, the transformant was transplanted into a medium (Amp L agar medium) prepared by adding 1.5 % of purified agar to the abovementioned Amp L medium. Thus colonies corresponding to a effieiency of 1.3 × 10⁶ were formed.

### (6) Colony hybridization (primary)

The cell suspension obtained in the step (5) was inoculated into the same L agar medium as the one used in the step (5) in 24 Petri dishes (14 × 10 cm) at a ratio of approximately 15000 colonies per dish and incubated until the diameter of each colony reached approximately 1 mm. A nylon membrane (Hybond-N, product of Amersham Co., Ltd.) was slowly placed thereon in such a manner that all colonies migrated to the membrane. Then said membrane was closely contacted with the surface of an L agar medium containing 250 µg/ml of chloramphenicol and incubated for two days and nights. The DNAs were fixed on the membrane in the following manner.

After the completion of the incubation, the membrane was treated with a denaturing solution (1.5 M NaCl and 0.5 M NaOH) for seven minutes and then neutralized by treating with a solution (1.5 M NaCl, 0.5 M Tris (pH 7.2) and 0.01 M EDTA) twice. Then it was washed with 6 × SSC (1 × SSC: 0.15 M NaCl and 0.015 M sodium citrate), air-dried and irradiated with UV light to thereby fix the DNAs on the membrane.

### (a) Prehybridization

The above membrane was heated to 65°C in a prehybridization solution comprising 6 × SSC, 0.1 % of BSA, 0.1 % of Ficoll, 0.1 % of polyvinylpyrrolidone, 0.5 % of SDS and 20 µg/ml of denatured herring sperm for three hours.

### (b) Hybridization

Subsequently the membrane was heated to 50°C in a hybridization solution comprising 175 µCi/µg DNA of the prehybridization solution of the probe as prepared in the step (1) and 10⁷ cpm/ml of the prehybridization solution for 16 hours.

### (c) Isolation of plasmid having base sequence coding hGSH·Px polypeptide

The membrane was washed with a 6 × SSC solution thrice, i.e., at 57°C for 30, 20 and 15 minutes. After air-drying, the membrane was subjected to autoradiography with the use of an X-ray film (Kodak XAR5). Thus 52 positive colony groups were selected.

### (7) Colony hybridization (secondary)

The colony groups obtained up to the step (6) were monocloned in an Amp L agar medium and the procedure of the step (6) was repeated. Thus four plasmids coding the hGSH·Px polypeptide were selected and the longest one was named pHGP 7.

### (8) Restriction map of cDNA coding hGSH·Px and determination of base sequence thereof

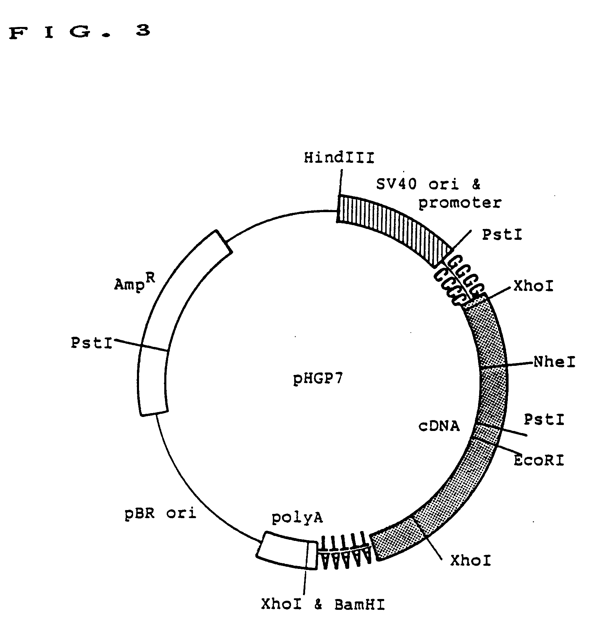

A DNA coding hGSH·Px was obtained by decomposing the pHGP 7 plasmid DNA with BamHI and then partially decomposing the same with PstI. A restriction map was prepared by further using XhoI, ECoRI and PstI. Thus it was proved that the pHGP 7 had a structure as shown in Fig. 3. It was proved that the 1134 bp DNA inserted into this plasmid coded hGSH·Px by determining the total base sequence of the inserted DNA by M 13 dideoxy method.

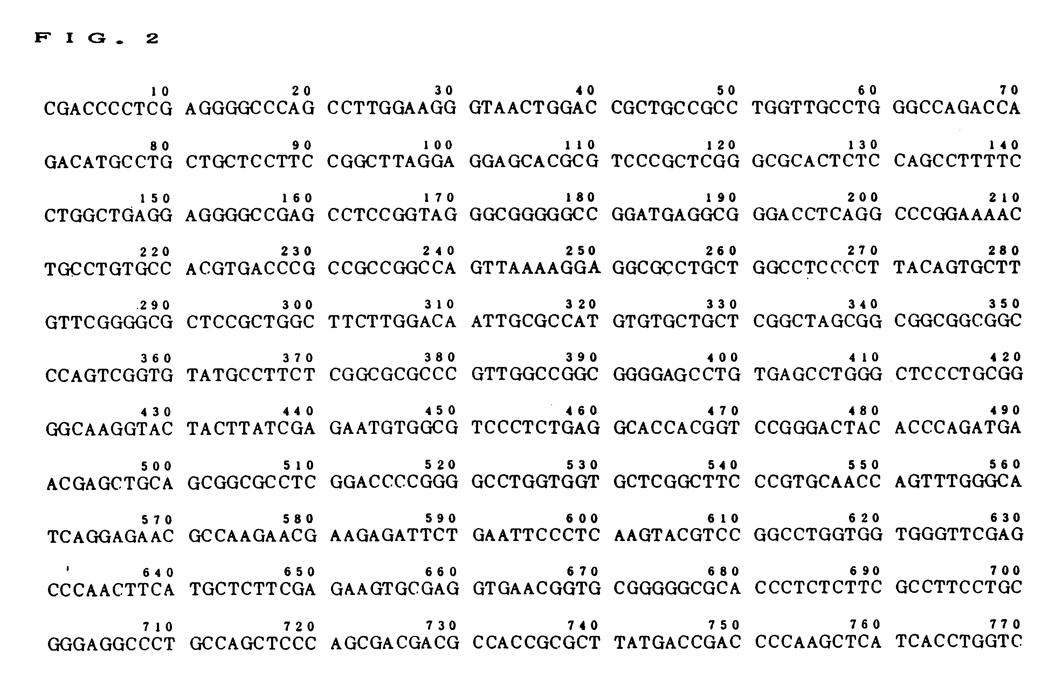

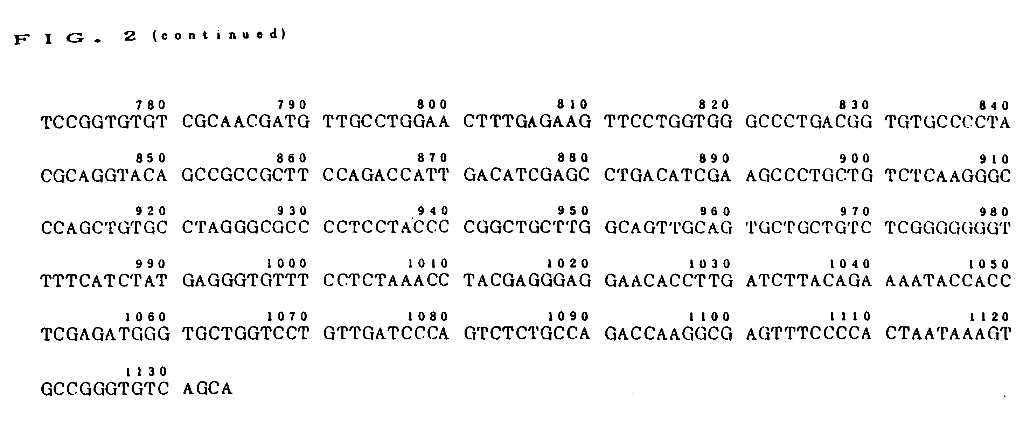

Fig. 2 shows the total base sequence, wherein the 319th to 321st base sequence ATG is an initiator codon; the 322nd to 921st one is a translation region; and the 922nd to 924th TAG is a terminator codon.

### (9) Determination of the activity of glutathione peroxidase

The activity of GSH·Px was determined by adding an appropriate amount of a GSH·Px solution to 3.0 ml of a reaction solution comprising 3 µmol of cumene hydroperoxide, 3 µmol of glutathione (reduced type), 150 µmol of a phosphate buffer (pH 7.0), 3 µmol of EDTA, 3 µmol of sodium azide, 0.64 µmol of NADPH and 3 U of glutathione reductase, heating the resulting mixture to 25°C and measuring a decrease in the absorbance thereof at 340 nm.

The amount of the enzyme capable of oxidizing 1 µmol of glutathione within one minute is defined as one unit of the GSH·Px activity.

### (10) Gene expression in COS cell

The pHGP 7 DNA obtained up to the step (7) was introduced into a COS 7 cell by calcium phosphate method (DNA Cloning, Vol. II, IRL Press, 152 - 153 (1985)) and the recombinant hGSH·Px activity in the cell was determined according to the method described in the step (9). Thus the activity thereof was found to be 1 U/ml of the incubation medium.

### (11) Gene expression in Xenopus laevis oocyte

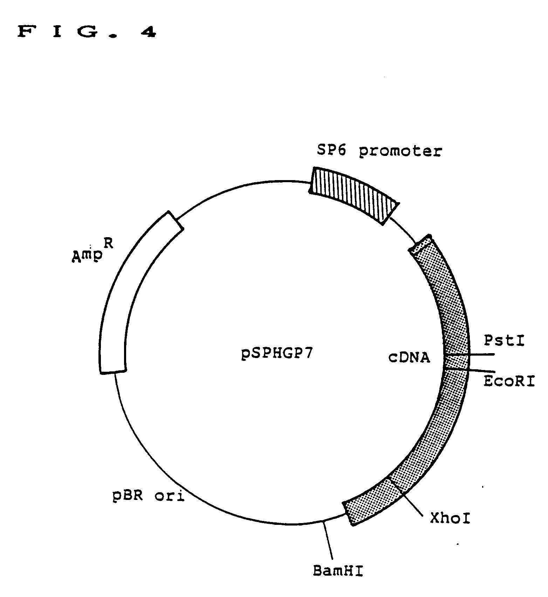

A DNA coding the hGSH·Px polypeptide was excised by decomposing the pHGP 7 plasmid DNA with BamHI and XhoI and then inserted into the Sal, BamHI site of pSP64 plasmid (a product of Amersham). The plasmid thus formed was named pSPHGP 7. Fig. 4 shows the structure thereof.

This pSPHGP 7 plasmid was decomposed with BamHI to give a straight-chain double-stranded DNA, which was then transcripted to an RNA in the presence of SP6 RNA polymerase (a product of BRL), ATP, GTP, CTP, UTP and m⁷G(5ʹ)p.p.p(5ʹ)G to thereby synthesize an mRNA.

A trace amount (40 - 50 pg/oocyte) of the mRNA thus obtained was injected into Xenopus laevis oocytes and these oocytes were maintained at 20°C for two days. Then the oocytes were broken and the GSH·Px activity of the supernatant was determined according to the method described in the step (9). The activity thus determined was 0.5 U/oocyte.

### (12) Structure of vector manifesting in E. coli cell

The plasmid pHGP 7 containing hGSH·Px as obtained in the step (7) was incised with BamHI and Nhe I to thereby give a DNA fragment of approximately 1 kbp. The following two single-stranded DNA were phosphorylated to the DNA fragment obtained above and then ligated by annealing:
single-stranded DNA No. 1:
(5ʹ)-GATCCATGTGTGCTGCTCGG-(3ʹ); and
single-stranded DNA No. 2:
(5ʹ)-CTAGCCGAGCAGCACACATG-(3ʹ).

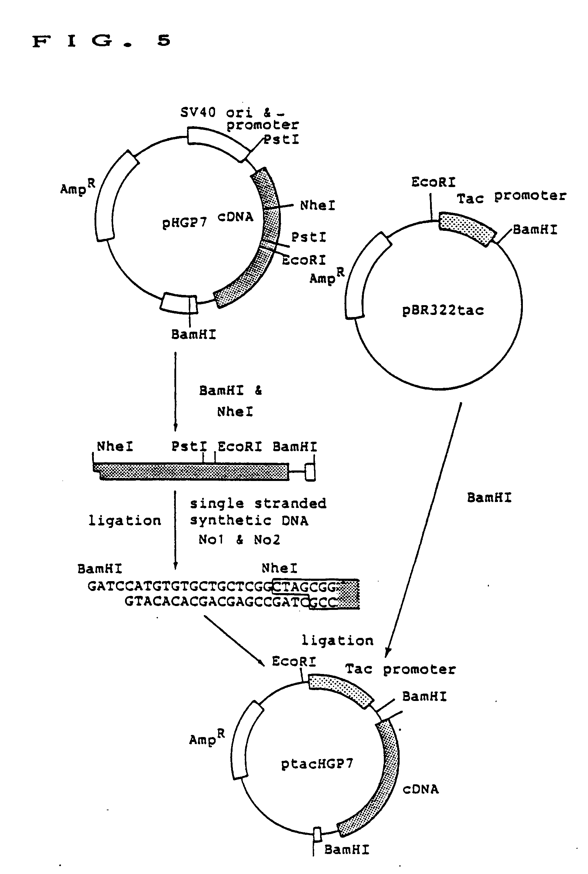

Then the product was incised with BamHI and inserted into the BamHI site of pBR322 tac plasmid DNA which was obtained by inserting a fragment of 121 bp containing Tac promoter of pDR540 (a product of Pharmacia) between the ECoRI and BamHI sites of pBR322. The plasmid thus obtained was named ptacHGP 7. Fig. 5 shows a method for preparing the same and the structure thereof.

### (13) Gene expression of hGSH·Px in E. coli

The ptacHGP 7 prepared in the step (12) was transformed into E. coli JM105 carrying lac I^{q} (a product of Takara Shuzo Co., Ltd.). The colonies thus obtained were incubated in a medium comprising 50 mM of Amp, 10 g/l of bactotrypton, 5 g/l of yeast extract and 5 g/l of sodium chloride at 37°C until the absorbance of the incubation medium at 600 nm reached 0.1 to 0.3. Then isopropyl-β-thiogalactoside was added thereto to give a final concentration of 1 mM and the incubation was carried out at 37°C for additional several hours. Then the incubation medium was centrifuged to thereby collect cells. These cells were broken by ultrasonication and the hGSH·Px activity of the supernatant was determined by the method described in the step (9). The activity thus determined was 5.5 U/ml of medium. This supernatant was lyophilized to thereby give a powder of recombinant hGSH·Px.

Alternately the aimed recombinant hGSH·Px may be prepared with the use of a lac I^{q} - free E. coli strain, such as C600 or N99cI⁺. For example, N99cI⁺ produced hGSH·Px at a yield of 1 mg/l or above in the presence of selenous acid.

## Claims

1. A DNA coding the amino acid sequence of recombinant human glutathione peroxidase, which has the base sequence as shown in Fig. 1.

2. A plasmid having a DNA coding the amino acid sequence of recombinant human glutathione peroxidase, wherein the base sequence of said DNA is the one as shown in Fig. 1.

3. A plasmid as set forth in Claim 2, which has a DNA coding the amino acid sequence of recombinant human glutathione peroxidase on the downstream side of a promoter, wherein the base sequence of said DNA is the one as shown in Fig. 1.

4. A process for preparing recombinant human glutathione peroxidase in a recombination host, which comprises the use of a DNA having the base sequence of claim 1.

5. The process of claim 4, wherein the DNA is a plasmid as set forth in any of claims 2 and 3.

6. The process according to one of claims 4 and 5, wherein the recombination host is E. coli.

## Patentansprüche

1. DNA, codierend für die Aminosäuresequenz von rekombinanter Human-Glutathionperoxidase, die die Basensequenz gemäß Fig. 1 aufweist.

2. Plasmid mit einer DNA, codierend für die Aminosäuresequenz von rekombinanter Human-Glutathionperoxidase, worin die Basensequenz der DNA eine solche ist, wie sie in Fig. 1 aufgeführt ist.

3. Plasmid nach Anspruch 2, das eine DNA hat, die für die Aminosäuresequenz der rekombinanter Human-Glutathionperoxidase codiert an der Stromabwärts-Seite eines Promoters, worin die Basensequenz der DNA eine ist, wie sie in Fig. 1 aufgeführt ist.

4. Verfahren zur Herstellung von rekombinanter Human-Glutathionperoxidase in einem Rekombinationswirt, welches umfaßt die Verwendung einer DNA mit der Basensequenz von Anspruch 1.

5. Verfahren nach Anspruch 4, worin die DNA ein Plasmid ist gemäß einem der Ansprüche 2 und 3.

6. Verfahren nach einem der Ansprüche 4 und 5, worin der Rekombinationswirt E. coli ist.

## Revendications

1. ADN codant la séquence d'acides aminés de la glutathion peroxydase humaine recombinante, qui a la séquence de base représentée dans la figure 1.

2. Plasmide ayant un ADN codant la séquence d'acides aminés de la glutathion peroxydase humaine recombinante, où la séquence de base dudit ADN est celle qui est représentée dans la figure 1.

3. Plasmide selon la revendication 2, qui a un ADN codant la séquence d'acides aminés de la glutathion peroxydase humaine recombinante du côté aval d'un promoteur, où la séquence de base dudit ADN est celle qui est réprésentée dans la figure 1.

4. Procédé de préparation de glutathion peroxydase humaine recombinante chez un hôte de recombinaison, comprenant l'utilisation d'un ADN ayant la séquence de base de la revendication 1.

5. Procédé de la revendication 4, dans lequel l'ADN est un plasmide selon l'une quelconque des revendications 2 et 3.

6. Procédé selon l'une des revendications 4 et 5, dans lequel l'hôte de recombinaison est E. coli.
